Europäisches Patentamt

European Patent Office

Office européen des brevets

(11) Veröffentlichungsnummer: **0 217 155 B1**

(19)

# (12) EUROPÄISCHE PATENTSCHRIFT

(45) Veröffentlichungstag der Patentschrift: **12.06.91**

(51) Int. Cl.⁵: **G01N 1/20**

(21) Anmeldenummer: **86112086.3**

(22) Anmeldetag: **02.09.86**

(54) **Verfahren und Vorrichtung zum Erstellen einer für ein Milchvolumen repräsentativen Milchprobe.**

(30) Priorität: **02.10.85 DE 3535179**

(43) Veröffentlichungstag der Anmeldung:
**08.04.87 Patentblatt 87/15**

(45) Bekanntmachung des Hinweises auf die
Patenterteilung:
**12.06.91 Patentblatt 91/24**

(84) Benannte Vertragsstaaten:
**AT BE CH DE FR IT LI NL SE**

(56) Entgegenhaltungen:
**EP-A- 0 098 966**
**DE-A- 2 453 473**
**DE-C- 3 439 663**
**GB-A- 2 088 332**
**US-A- 1 964 775**

(73) Patentinhaber: **Jansky Milchsammelwagen-
und Apparatebau GmbH
Taubenstrasse 25-43
W-4407 Emsdetten(DE)**

(72) Erfinder: **Jansky, Manfred, Dipl.-Ing.
Taubenstrasse 37
W-4407 Emsdetten(DE)**
Erfinder: **Jansky, Walter
Kapellenweg 11
W-4407 Emsdetten(DE)**

(74) Vertreter: **Patentanwaltsbüro Cohausz & Florack
Schumannstrasse 97
W-4000 Düsseldorf 1(DE)**

**Beschreibung**

Es ist üblich, mit Milchsammelwagen Milch unterschiedlicher Volumina und unterschiedlicher Qualität in Empfang zu nehmen. Die Milchsammelwagen sind deshalb mit einem Volumenmesser und einer Probeentnahmevorrichtung ausgerüstet, die es ermöglicht, mehr oder weniger exakt eine repräsentative Probe für die vom jeweiligen Produktionsbetrieb abzuliefernde Milch zu erstellen. Dies geschieht gemäß z.B. EP-A-98966 in der Weise, daß während des gesamten Annahmevorganges ein Teilstrom aus dem Hauptstrom abgezweigt und in einen kleinen Sammel- und Mischbehälter gefüllt wird, aus dem am Ende des Annahmevorganges ein noch kleineres Volumen als Milchprobe entnommen wird. Nach Erstellen der Probe wird die übrige im Sammel- und Mischbehälter verbleibende Milch abgelassen und der bereits angenommenen Milch zugegeben. Beim nächsten Produzenten wird in gleicher Weise verfahren, also der Sammel- und Mischbehälter während des Annahmevorganges mengenproportional mit Milch gefüllt. Da von der letzten Annahme im Behälter eine Benetzung geblieben ist, wird diese neue in den Sammel- und Mischbehälter gefüllte Milch mit der die Behälterwandung benetzenden Milch aus der letzten Annahme vermischt. Wenn auch dieses Volumen klein ist, kann es zu einer beachtlichen Verfälschung der beim jeweils folgenden Produzenten angenommenen Milchprobe führen. Diese Gefahr der Probenverfälschung ist umso größer, je kleiner das Volumen der in den Sammel- und Mischbehälter eingefüllten Milchmenge ist. Um diese Verfälschung möglichst klein zu halten ist es bekannt, das Abzweigventil so einzustellen, daß unter Berücksichtigung des jeweiligen, abzunehmenden Milchvolumens der Proportionalitätsfaktor für die abzuzweigende Milch größer oder kleiner gewählt wird. Diese Art der Erstellung der Probe ist nicht nur umständlich, sondern nach wie vor ungenau, denn selbst eine erfahrene Bedienungsperson wird beim Abschätzen des abzunehmenden Milchvolumens kaum jedes Mal den Proportionalitätsfaktor am Abzweigventil so einstellen können, daß der Sammel- und Mischbehälter unter optimaler Ausnutzung seiner Kapazität während des gesamten Annahmevorganges mit abgezweigter Milch gefüllt wird, ohne daß er am Ende des Annahmevorganges überfüllt wird. Weiter ist es aus der EP-A-98966 bekannt, etwa noch an den Wandungen anhaftende Restmilchmengen mittels einer Durchspülung zu entfernen, bevor die Milchprobe genommen wird.

Bei einem Verfahren und einer Vorrichtung zum Erstellen einer für ein Milchvolumen repräsentativen Milchprobe, das bzw. die Gegenstände der am 21.11.85 veröffentlichten Patentanmeldung EP-A-161552 sind, wird von dem von einem ersten Behälter zu einem zweiten Behälter führenden Hauptleitung ein Teilstrom mit einem bestimmten Proportionalitätsfaktor abgezweigt. Das Abzweigen erfolgt dabei in zwei zeitlich aufeinanderfolgenden Phasen, wobei in der ersten Phase der Proportionalitätsfaktor wesentlich größer als in der zweiten Phase ist.

Ist das insgesamt vom Lieferanten anzunehmende Milchvolumen klein, reicht die Kapazität des Probeentnahmebehälters aus, den mengenproportionalen Teilstrom während des gesamten Annahmevorganges anzunehmen. Ist die anzunehmende Milchmenge dagegen groß, müssen beide Annahmephasen durchlaufen werden. Aus dem ersten Teilvolumen mit dem größeren Proportionalitätsfaktor wird nach der ersten Phase nur ein solcher Teil zurückbehalten, wie er dem kleineren Proportionalitätsfaktor in der zweiten Phase entspricht. Beide Teilmengen lassen sich dann anschließend vermischen und geben die repräsentative Milchprobe ab. Die Genauigkeit, mit der das erste Teilvolumen auf die gewünschte Restmenge reduziert wird, die dem Proportionalitätsfaktor in der zweiten Annahmephase entspricht, ist bestimmend für die Genauigkeit der aus diesem Restvolumen und dem in der zweiten Phase angenommenen Volumen. Beim Gegenstand der älteren Anmeldung wird das Teilvolumen durch einen Überlauf in dem Probeentnahmebehälter bestimmt. Bei Schieflage des Probeentnahmebehälters kann es deshalb zu Ungenauigkeiten kommen.

Vor dem Hintergrund dieses Standes der Technik liegt der Erfindung die Aufgabe zugrunde, ein Verfahren und eine Vorrichtung zu schaffen, mit der eine sehr exakte repräsentative Milchprobe sowohl für große als auch kleine Volumina zu erstellen ist.

Die Erfindung bezieht sich auf ein Verfahren zum Erstellen einer für ein Milchvolumen repräsentativen Milchprobe, bei dem während des Umfüllens der Milch von einem ersten Behälter in einen zweiten Behälter mengenproportionale Teilströme mit verschieden großen Proportionalitätsfaktoren aus dem Hauptstrom vom ersten Behälter in den zweiten Behälter abgezweigt und in Probeentnahmebehältern gesammelt werden, wobei nur bei Überschreiten der Kapazität des mit großem Proportionalitätsfaktor gefüllten Probeentnahmebehälters das Abzweigen in zwei zeitlich aufeinanderfolgenden Phasen mit zunächst großem und dann wesentlich kleinerem Proporitonalitätsfaktor erfolgt. Bei einem solchen Verfahren wird gemäß der Erfindung in der ersten Phase parallel zu dem in den einen Probeentnahmebehälter abgezweigten Teilstrom mit dem großen Proportionalitätsfaktor ein zweiter Teilstrom mit dem kleineren Proportionalitätsfaktor in den anderen Probeentnahmebehälter abgezweigt, und das in der ersten Phase mit dem kleinen Proportionalitätsfaktor gesammelte Teilvolumen mit dem in der zweiten Phase mit dem gleichen kleinen Proportionalitätsfaktor gesammelten Teilvolumen vermischt.

Gegenstand der Erfindung ist ferner eine Probeentnahmevorrichtung für eine Milchumfüllanlage mit einer von einem ersten Behälter zu einem zweiten Behälter führenden Hauptleitung und zwei davon mit einem kleinen bzw. großen Teilungsverhältnis abzweigenden und zu Probeentnahmebehältern führenden Abzweigleitungen, wobei ein Abzweigventil zum Einstellen der Teilungsverhältnisse der abgezweigten Milch vorgesehen ist, das bei Erreichen eines vorgegebenen Niveaus in dem mit dem großen Teilungsverhältnis gefüllten Probeentnahmebehälter über eine erste Servo-Steuerung die zu diesem Probeentnahmebehälter führenden Leitung von dem großen Teilungsverhältnis auf das kleine Teilungsverhältnis umschaltet, und das Entleeren der gesammelten Milch aus diesem Probeentnahmebehälter einleitet, und wobei der andere Probeentnahmebehälter, der über das Abzweigventil bei dem eingestellten kleinen Teilungsverhältnis füllbar ist, in den einen Probeentnahmebehälter entleerbar ist. Bei einer solchen Probeentnahmevorrichtung ist gemäß der Erfindung das Abzweigventil so konstruiert, daß beide Probeentnahmebehälter gleichzeitig mit den verschieden eingestellten Teilungsverhältnissen füllbar sind, und der eine Probeentnahmebehälter über ein von einer zweiten Servo-Steuerung bei Erreichen des vorgegebenen Niveaus gesteuertes Ventil ganz entleerbar ist, bevor die in dem anderen Probeentnahmebehälter gesammelte Milch in den einen Probeentnahmebehälter entleert wird.

Da bei der Erfindung vorsorglich bereits in der ersten Phase neben der mit großen Proportionalitätsfaktor abgezweigten Milch,Milch mit wesentlich kleinerem Proportionalitätsfaktor abgezweigt und gesammelt wird, ist es nicht erforderlich, von der mit großem Proportionalitätsfaktor abgezweigten Milch ein Restvolumen dann zurückzuhalten, wenn die Kapazität des mit großem Proportionalitätsfaktor gefüllten Probeentnahmebehälters nicht ausreicht, während der gesamten Annahmephase die abgezweigte Milch mit großem Proportionalitätsfaktor anzunehmen. In diesem Fall wird die mit dem kleinen Proportionalitätsfaktor abgezweigte Milch mit dem in der zweiten Phase mit kleinem Proportionalitätsfaktor abgezweigten Milch vermischt, um daraus die für die gesamte Annahme repräsentative Milchprobe erstellen zu können. Ist jedoch das insgesamt anzunehmende Milchvolumen nicht groß, so daß während der gesamten Annahme Milch mit großem Proportionalitätsfaktor abgezweigt und in dem zugeordneten Behälter aufgenommen werden kann, wird daraus die Milchprobe entnommen, die mit dem kleinen Proportionalitätsfaktor gesammelte Milch wird in diesem Fall für die Erstellung der Probe nicht benötigt. Die durch die Lage der Vorrichtung sonst auftretenden Ungenauigkeiten bei der Bildung aus dem ersten Teilvolumen abgeleiteten Restvolumens treten also nicht auf.

Im folgenden wird die Erfindung anhand einer ein Ausführungsbeispiel darstellenden Zeichnung näher erläutert.

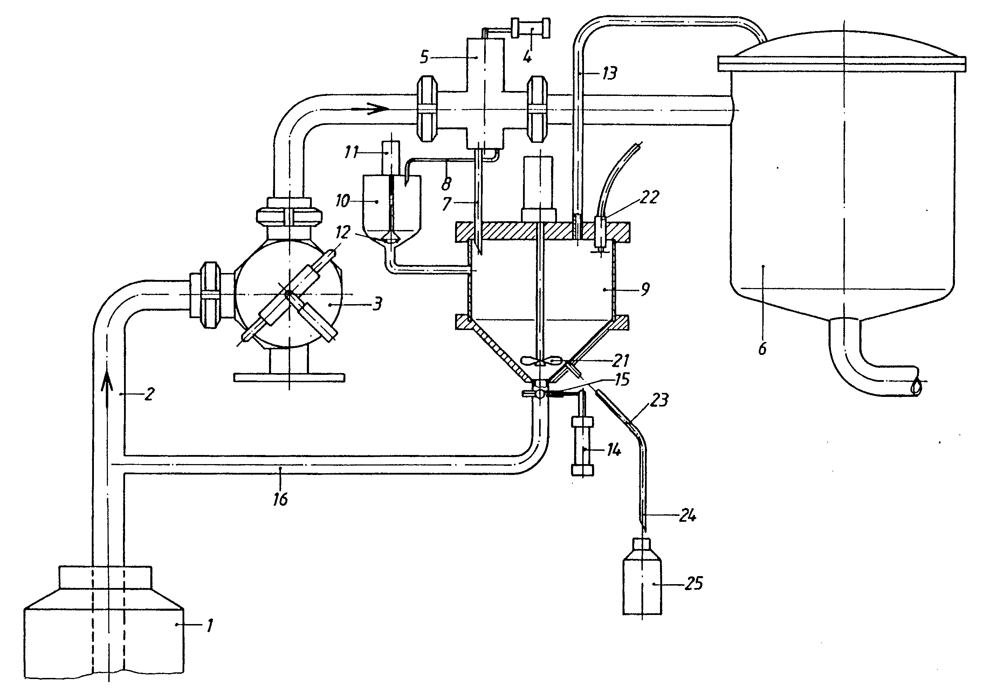

Gemäß dem Ausführungsbeispiel der Zeichnung wird die von einem Produzenten abzuliefernde Milch in Behältern 1 zum Abholen durch einen Milchsammelwagen bereitgestellt. Über eine Hauptleitung 2, eine selbst ansaugende Pumpe 3 und ein mittels einer Servo-Betätigung 4 auf zwei unterschiedliche Teilungsverhältnisse einstellbares Ventil 5 wird die Milch in einen Behälter 6 (Luftabscheider) gefördert, aus dem die Milch in einen nicht dargestellten Sammeltank des ebenfalls nicht dargestellten Tankfahrzeuges gelangt.

Von dem Abzweigventil 5 zweigen zwei Abzweigleitungen 7,8 in einen größeren und einen kleineren Probeentnahmebehälter 9,10 ab, wobei die einem großen Teilungsverhältnis zugeordnete Abzweigleitung 7 unmittelbar in den größeren Behälter 9 und die einem kleinen Teilungsverhältnis zugeordnete Abzweigleitung 8 in den kleineren Behälter 9 mündet. Der kleinere Behälter 10 ist über ein mit einer Servo-Betätigung 11 ausgestattetes Ventil 12 am Boden des Behälters 10 in den Behälter 9 entleerbar.

Der Behälter 9 ist über eine Entlüftungsleitung 13 mit dem Behälter 6 verbunden. Der Inhalt des Behälters 9 kann über ein mit einer Servo-Betätigung 14 betätigbares Ventil 15 abgelassen und über eine Rücklaufleitung 16 in die Hauptleitung eingespeist werden. Mit einem im Behälter angeordneten Rührwerk 21 kann die Milch im Behälter 9 gemischt werden.

Zur Aktivierung der verschiedenen Servo-Betätigungen 4,11,14,18 dient eine nicht dargestellte Steuereinrichtung, die von einem Niveaustandsfühler 22 ein Signal erhält, sobald der Milchpegel im Behälter 9 den Fühler 22 erreicht.

Zur Entnahme einer Milchprobe ist am unteren Bereich des Behälters eine Leitung 23 angeschlossen, die mit einer Hohlnadel 24 in ein Probegefäß 25 einführbar ist. Damit in das Probegefäß 25 Milch gelangen kann, ist zum Beispiel eine weitere nicht dargestellte Hohlnadel vorgesehen, die mit der Unterdruckseite der Pumpe 3 verbunden ist. Sobald beide Nadeln 24 einen abdichtenden Deckel des Probegefäßes 25 durchstoßen haben, gelangt die zu entnehmende Milchprobe aufgrund des Unterdruckes in das Gefäß 25.

Die beschriebene Probeentnahmevorrichtung arbeitet auf folgende Art und Weise:

Nach jedem Annahmevorgang und Entnahme der Probe wird das Ventil 15 geöffnet, so daß aus dem Behälter 9 die gesamte Milch fließen kann. An der Behälterwandung bleibt nur eine Benetzung übrig. Zur Übernahme neuer Milch wird die Hauptleitung 2 mit ihrem unten offenen Ende in den Behälter 1 gesteckt und die Pumpe 3 fördert Milch aus dem Behälter 1 in den Behälter 6. Während dieser Phase ist das Ventil 5 für die Leitung 7 auf ein großes Teilungsverhältnis und für die Leitung 8 auf ein wesentliches kleineres Teilungsverhältnis eingestellt, beispielsweise auf die Teilungsverhältnisse 10 % und 3 o/oo. Erreicht der Milchpegel im Behälter 9 während dieser Phase der Annahme den Fühler 22, dann ist die erste Phase der Annahme beendet. Die Steuereinrichtung veranlaßt über die Servo-Steuerung 4 das Ventil 5, die Abzweigung von Milch mit großem Teilungsverhältnis zu sperren und nur noch Milch mit dem kleinen Teilungsverhältnis abzuzweigen. Dies geschieht entweder dadurch, daß nur noch Milch über die Leitung 8 in den Behälter 10 gelangt oder aber über die Leitung 7 Milch mit dem kleineren Teilungsverhältnis, also beispielsweise 3 o/oo, direkt in den Behälter 9. Beim Ansprechen des Fühlers 22 veranlaßt die Steuereinrichtung die Servo-Betätigung 14 das Ventil 15 zu öffnen, so daß die gesamte mit dem größeren Proportionalitätsfaktor gesammelte Milch aus dem Behälter 9 abfließt. Nach Entleeren des Behälters 9 wird das Ventil 15 wieder geschlossen und jetzt gelangt nur noch Milch mit dem kleineren Proportionalitätsfaktor in den Behälter 9, sei es direkt über die Leitung 7, sei es indirekt über den Behälter 10. Auch die in der ersten Phase im Behälter 10 gesammelte Milch wird nach Öffnen des Ventiles 12 in den Behälter 9 gefüllt und durch das Rührwerk 20 mit der in der zweiten Phase angenommenen Milch vermischt. Auf diese Art und Weise erhält man am Ende der zweiten Phase ein Teilvolumen, das repräsentativ für die gesamten Annahmevorganges angenommene Milch ist.

Sofern in der ersten Phase das Niveau des Fühlers 22 nicht erreicht wird, wird die Milchprobe nur aus dem im Behälter 9 befindlichen Milchvolumen genommen. Die mit dem kleineren Proportionalitätsfaktor im Behälter 10 gesammelte Milch wird für die Probeentnahme dann nicht gebraucht.

**Ansprüche**

1. Verfahren zum Erstellen einer für ein Milchvolumen repräsentativen Milchprobe, bei dem während des Umfüllens der Milch von einem ersten Behälter in einen zweiten Behälter mengenproportionale Teilströme mit verschieden großen Proportionalitätsfaktoren aus dem Hauptstrom vom ersten Behälter in den zweiten Behälter abgezweigt und in Probeentnahmebehältern gesammelt werden, wobei nur bei Überschreiten der Kapazität des mit dem großen Proportionalitätsfaktor gefüllten Probeentnahmebehälters das Abzweigen in zwei zeitlich aufeinanderfolgenden Phasen mit zunächst großem und dann wesentlich kleinerem Proportionalitätsfaktor erfolgt, wobei in der ersten Phase parallel zu dem in den einen Probeentnahmebehälter abgezweigten Teilstrom mit dem großen Proportionalitätsfaktor ein zweiter Teilstrom mit dem kleinen Proportionalitätsfaktor in den anderen Probeentnahmebehälter abgezweigt wird und das in der ersten Phase mit dem kleinen Proportionalitätsfaktor gesammelte Teilvolumen mit dem in der zweiten Phase mit dem gleichen kleinen Proportionalitätsfaktor gesammelten Teilvolumen vermischt wird.

2. Probeentnahmevorrichtung für eine Milchumfüllanlage mit einer von einem ersten Behälter (1) zu einem zweiten Behälter (6) führenden Hauptleitung (2) und zwei davon mit einem kleinen bzw. großen Teilungsverhältnis abzweigenden und zu Probeentnahmebehältern (9,10) führenden Abzweigleitungen (7,8), wobei zur Einstellung der Teilungsverhältnisse der abgezweigten Milch ein Abzweigventil (5) vorgesehen ist, das bei Erreichen eines vorgegebenen Milchniveaus in dem mit dem großen Teilungsverhältnis gefüllten Probeentnahmebehälter (9) über eine erste Servo-Steuerung (4) die zu diesem Probeentnahmebehälter führenden Abzweigleitung (7) von dem großen Teilungsverhältnis auf das kleine Teilungsverhältnis umschaltet, und das Entleeren der gesammelten Milch aus diesem Behälter (9) einleitet, und wobei der andere Probeentnahmebehälter (10), der über das Abzweigventil (5) bei dem eingestellten kleinen Teilungsverhältnis füllbar ist, in den einen Probeentnahmebehälter (9) entleerbar ist, wobei das Abzweigventil (5) so konstruiert ist, daß beide Probeentnahmebehälter (9,10) gleichzeitig bei den verschieden großen eingestellten Teilungsverhältnissen füllbar sind und wobei der eine Probeentnahmebehälter (9) über ein von einer zweiten Servo-Steuerung (14) bei Erreichen des vorgegebenen Niveaus gesteuertes Ventil (15) ganz entleerbar ist, bevor die in dem anderen Probeentnahmebehälter (10) gesammelte Milch in den einen Probeentnahmebehälter (9) entleerbar ist.

**Claims**

1. A method of preparing a milk sample representative of a volume of milk, wherein during the transfer of the milk from a first tank to a second tank component flows of proportional quantity with different proportionality factors are branched off from the first tank to the second tank and collected in sampling tanks, the branching-off into two phases succeeding one another in time, first with a high and then with a substantially lower proportionality factor taking place only when the capacity of the sampling tank filled with the high proportionality factor is exceeded, while in the first phase, parallel with the component flow having the high proportionality factor branched off to one sampling tank, a second component flow with the low proportionality factor is branched off to the other sampling tank, and the component volume collected in the first phase with the low proportionality factor is mixed with the component volume collected in the second phase with the same low proportionality factor.

2. A sampling apparatus for a milk transfer installation having a main pipe (2) extending from a first tank (1) to a second tank (6), and two branch pipes (7, 8) branching off therefrom with a low and high proportionality factor respectively and extending to sampling tanks (9, 10), while for the adjustment of the proportionality factors of the branched-off milk a branch valve (5) is provided which, when a predetermined level of milk is reached in the sampling tank (9) filled with the high proportionality factor, switches over via a first servo control system (4) the branch line (7) extending to said sampling tank from the high proportionality factor to the low proportionality factor and initiates the emptying of the collected milk from said tank (9), the other sampling tank (10), which can be filled via the branch valve (5) with the adjusted low proportionality factor, being emptyable into a sampling tank (9), the branch valve (5) being so constructed that the two sampling tanks (9, 10) can be filled simultaneously with the proportionality factors adjusted to different values, one sampling tank (9) being completely emptyable via a valve (15) controlled by a second servo control system (14) when the predetermined level is reached, before the milk collected in the other sampling tank (10) can be emptied into said sampling tank (9).

## Revendications

1. Procédé pour prélever un échantillon de lait représentatif d'un volume de lait dans lequel, pendant le transvasement du lait d'un premier dans un second réservoir, des débits partiels du débit principal proportionnel à la quantité sont dérivés, avec des facteurs de proportionnalité de grandeurs différentes du courant principal du premier réservoir dans le second réservoir et sont collectés dans des réservoirs de prélèvement d'échantillons, la dérivation en deux phases se succédant l'une à l'autre dans le temps avec d'abord un facteur de proportionnalité élevé et ensuite considérablement plus faible ne se produisant que dans le cas d'un dépassement de la capacité du réservoir de prélèvement d'échantillons rempli avec le facteur de proportionnalité élevé, un second courant partiel avec le faible facteur de proportionnalité étant dérivé dans l'autre réservoir de prélèvement d'échantillons dans la première phase en parallèle avec le courant partiel avec le facteur de proportionnalité élevé dérivé dans un premier réservoir de prélèvement d'échantillons, et le volume partiel rassemblé avec le facteur de proportionnalité faible dans la première phase étant mélangé au volume partiel rassemblé avec le même facteur de proportionnalité faible dans la seconde phase.

2. Dispositif de prélèvement d'échantillons pour un dispositif collecteur de lait, avec une conduite principale (2) menant d'un premier réservoir (1) à un second réservoir (6) et deux conduites de dérivation (7,8) qui en dérivent avec un rapport de division faible, respectivement grand, et conduisent à des réservoirs de prélèvement d'échantillons (9,10), une vanne de dérivation (5) étant prévue pour le réglage du rapport de division du lait dérivé qui, lors de l'arrivée à un niveau de lait prédéterminé dans le réservoir (10) rempli avec le rapport de dérivation élevé, commute par une première servocommande (4) la conduite de dérivation (7) conduisant à ce réservoir de prélèvement d'échantillons du rapport de division élevé au rapport de division faible, et commence le vidage du lait rassemblé hors de ce réservoir (9), et l'autre réservoir de prélèvement d'échantillons (10), qui peut être rempli par l'intermédiaire de la vanne de dérivation (5) avec le rapport de division faible réglé, pouvant être vidé dans un réservoir de prélèvement d'échantillons (9), la vanne de dérivation (5) étant construite de telle manière que les deux réservoirs de prélèvement d'échantillons (9,10) peuvent être remplis simultanément par les rapports de division réglés à des grandeurs différentes, et l'un des réservoirs de prélèvement d'échantillons (9) pouvant être totalement vidé par l'intermédiaire d'une vanne (15) commandée par une seconde servo-commande (14) lors de l'arrivée au niveau prédéterminé, avant que le lait rassemblé dans l'autre réservoir de prélévement d'échantillons (10) puisse être vidé dans le premier réservoir de prélèvement d'échantillons (9).